# EUROPÄISCHE PATENTSCHRIFT

(11) **EP 0 718 283 B1**
(45) Veröffentlichungstag und Bekanntmachung des Hinweises auf die Patenterteilung: **19.08.1998**
(21) Anmeldenummer: 95119979.3
(22) Anmeldetag: 18.12.1995
(51) Int. Cl.: C07C 403/14

(54) **Verfahren zur Herstellung von Retinal durch Oxidation von Retinol mit Sauerstoff in Gegenwart von 4-Hydroxy-2,2,6,6-tetramethyl-piperidin-1-oxyl und Kupfer(I)-chlorid**
Process for the preparation of retinal by oxidation of retinol with oxygen in the presence of 4-hydroxy-2,2,6,6-tetramethyl-piperidin-1-oxyl and copper(I)chloride
Procédé de préparation du rétinal par oxydation du rétinol par l'oxygène en présence de 4-hydroxy-2,2,6,6-tétraméthyl-pipéridin-1-oxyle et de chlorure cuivreux

(30) Priorität: 23.12.1994 DE 4446451
(43) Veröffentlichungstag der Anmeldung: 26.06.1996
(73) Patentinhaber: BASF AKTIENGESELLSCHAFT, 67056 Ludwigshafen (DE)
(72) Erfinder: Krause, Wolfgang, Dr., D-68782 Brühl (DE); Paust, Joachim, Dr., D-67141 Neuhofen (DE)

(56) Entgegenhaltungen:
- EP-A- 0 157 738
- EP-A- 0 282 760

## Beschreibung

Die Erfindung betrifft ein verbessertes Verfahren zur Herstellung von Retinal (Vitamin-A-Aldehyd) der Formel I 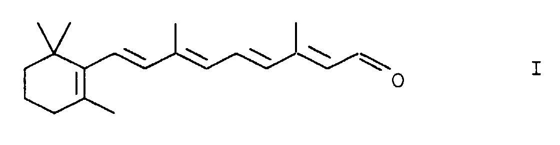 durch Oxidation von Retinol mit Sauerstoff in Gegenwart von 4-Hydroxy-2,2,6,6-tetramethyl-piperidin-1-oxyl und Kupfer(I)chlorid.

Retinal besitzt wertvolle biologische Aktivität bzw. ist ein Baustein zur Darstellung wichtiger Carotinoide. So kann aus Retinal durch Wittig-Olefinierung mit Retinyl-triphenylphosphoniumsalzen das begehrte β-Carotin hergestellt werden (DE 10 68 709). Ausgehend von Retinal sind zudem Synthesen der im Futter- und Lebensmittelsektor begehrten β-Apocarotinoide möglich (Pure Appl. Chem. (1991), S. 45-58 und Liebigs Ann. Chem. (1976), S. 2194-2205).

Für die Darstellung von Polyenaldehyden wie Retinal aus Polyenalkoholen sind verschiedene Methoden bekannt.

Die Überführung eines Polyenalkohols in einen Polyenaldehyd ist jedoch keine triviale Reaktion, da sowohl das Edukt als auch das Produkt sehr empfindlich sind (Synthesis (1976), S. 65 ff.).

Dementsprechend werden an das Oxidationsmittel sehr spezielle Anforderungen gestellt. Als geeignete Reagenzien zur Oxidation sind Metalloxide wie Mangandioxid oder Nickelperoxid beschrieben. Nachteile der zumeist heterogen eingesetzten Metalloxide sind die stark schwankenden Ausbeuten (Chem. Rev. 67 (1967), S. 188), der benötigte große Überschuß an Oxidationsmittel (J. Chem. Soc. (1952), S. 1094) und die Notwendigkeit der Aktivierung der Metalloxide. Als wirksames Oxidationssystem zur Oxidation von Polyenen ist auch schon die Sauerstoffoxidation in Gegenwart des Katalysatorsystems 2,2,6,6-Tetramethyl-piperidin-1-oxyl bzw. 4-Oxo-2,2,6,6-tetramethyl-piperidin-1-oxyl und Kupfer(I)-chlorid in DE-A 37 05 785 beschrieben. In den dort zitierten Beispielen 1 bis 5 sind für die Oxidation je 10 Mol-% Kupfer(I)-chlorid und 2,2, 6, 6-Tetramethyl-piperidin-1-oxyl bzw. 4-Oxo-2,2,6,6-Tetramethyl-piperidin-1-oxyl erforderlich. Auch gemäß Anspruch 6 ist eine Katalysatormenge von 5 bis 10 Mol-% bevorzugt. Trotz der relativ hohen Katalysatormenge betragen die Ausbeuten für isoliertes, kristallines Retinal aber nur bis zu 72,6 %.

Es wurde nun überraschenderweise gefunden, daß man Retinal mit wesentlich geringerer Katalysatormenge und mit deutlich höheren Ausbeuten gewinnen kann, wenn man Retinal in Gegenwart des Katalysatorsystems 4-Hydroxy-2,2,6,6-tetramethyl-piperidin-1-oxyl und Kupfer(I)-chlorid in einem Amidgruppen enthaltenden Lösungsmittel mit Sauerstoff oder einem Sauerstoff enthaltenden Gas oxidiert.

Man erhält Ausbeuten an reinem kristallinen Retinal von 80 bis 95 % bei einer all-E-Selektivität von 92 bis 99 %.

Die Ausgangsverbindung Retinol (Vitamin A-Alkohol) ist großtechnisch verfügbar als Zwischenstufe der Vitamin A-Acetat-Synthese.

Als Amidgruppen enthaltende Lösungsmittel kommen vorzugsweise N,N-Dimethylacetamid, 1-Methyl-2-pyrrolidon, N,N-Dimethylpropylenharnstoff oder N,N-Dimethylethylenharnstoff und vor allem N,N-Dimethylformamid in Betracht.

Die Herstellung von 4-Hydroxy-2,2,6,6-tetramethyl-piperidin-1-oxyl ist aus EP-B 0 157 738 bekannt.

Sowohl dieser Katalysator als auch der Co-Katalysator Cu(I)Cl werden in der Regel in Mengen von 2 bis 5 Mol-% angewandt. Größere Mengen schaden zwar nicht, jedoch werden durch Verwendung größerer Mengen die Ausbeuten nicht mehr erhöht. Das Verhältnis von 4-Hydroxy-2,2,6,6-tetramethyl-piperidin-1-oxyl zu CuCl beträgt in der Regel 1 : 1 bis 1 : 3.

Das Verfahren wird vorteilhafterweise so ausgeführt, daß Retinol zusammen mit dem Katalysatorsystem in dem Lösungsmittel, vorzugsweise in Dimethylformamid, vorgelegt wird und durch Eingasung von Sauerstoff oder sauerstoffenthaltenden Gasen oxidiert wird.

Die Reaktionstemperatur beträgt vorzugsweise 20 bis 40°C, besonders bevorzugt 25 bis 35°C. Die Reaktionszeiten betragen 0,5 bis 6 Stunden, bevorzugt 1 bis 4 Stunden. Auf die in DE-A 37 05 785 beschriebene aufwendige Aufarbeitungsmethode mit Entfernen des Katalysators durch Schütteln mit Natriumjodid-Lösung in Salzsäure und Chromatographie kann vorteilhafterweise verzichtet werden.

Wird für Folgeumsetzungen besonders Kupferionen-armes Retinal benötigt, ohne daß eine Molekular-Destillation durchgeführt werden muß, so kann mit besonderem Vorteil eine Waschung mit verdünnten wäßrigen Lösungen von Komplexbildnern, wie Natriumsalzen der Ethylendiamintetraessigsäure, erfolgen.

Die Oxidationsausbeuten betragen allgemein 95 bis 99 %; die Ausbeuten an reinem kristallinem Retinal betragen je nach Aufarbeitungsmethode 80 bis 95 %.

### Beispiel 1

### Synthese von Retinal

9,64 kg 95 %iges Retinol (32 Mol; all-E-Gehalt 96 %) wurden in 15 l Dimethylformamid gelöst. Dazu wurden 275,6 g 4-Hydroxy2,2,6,6-tetramethyl-piperidin-1-oxyl (1,6 Mol) und 158,4 g Kupfer(I)-Chlorid (1,6 Mol) hinzugefügt und innerhalb von 2 bis 3 Stunden wurde bei 20°C bis 35°C unter Rühren Sauerstoff eingegast (zwischen 500 und 2000 l/h).

Zur Aufarbeitung wurde die homogene Produktlösung mit Heptan, Hexan, Cyclohexan oder Toluol extrahiert.

Nach Waschen der organischen Phase mit Wasser oder 0,5 %igen wäßrigen Lösungen von Ethylendiamintetraacetat-Natriumsalzen wurde die organische Lösung von Retinal getrocknet und dann entweder direkt in Folgestufen umgesetzt (Ausbeute: 95 %, E/Z-Verhältnis 92/8) oder durch Kristallisation aus Hexan oder Heptan gereinigt. Ausbeute Kristallisat: 8,18 kg kristallines Retinal (90 %), all-E-Gehalt 99 %, UV-Wert E₁ = 1525 bei 381 nm (EtOH).

| Tabelle zu Beispiel 1: | | | | | |
|---|---|---|---|---|---|
| Retinal [Mol] | 4-OH-TEMPO [Mol] | CuCl [Mol] | Reaktionszeit [h] | Oxidationsausbeute [%] | Ausbeute [%, gereinigt] |
| 10 | 0,2 | 0,2 | 6 | 95 | 81 |
| 10 | 0,3 | 0,3 | 3 | 98 | 84 |
| 10 | 0,3 | 0,4 | 3 | >98 | 88 |
| 10 | 0,35 | 0,5 | 2 | >99 | 88 |
| 10 | 0,5 | 0,5 | 2 | >99 | 90 |

## Patentansprüche

1. Verfahren zur Herstellung von Retinal, dadurch gekennzeichnet, daß man Retinol in homogener Phase in einem Amidgruppen enthaltenden Lösungsmittel mit katalytischen Mengen 4-Hydroxy-2,2,6,6-tetramethyl-piperidin-1-oxyl und katalytischen Mengen Kupfer(I)-chlorid mit Sauerstoff oder sauerstoffhaltigen Gasen oxidiert.

2. Verfahren nach Anspruch 1, dadurch gekennzeichnet, daß man das 4-Hydroxy-2,2,6,6-tetramethyl-piperidin-1-oxyl in Mengen von 2 bis 5 Mol-%, bezogen auf Retinol einsetzt.

3. Verfahren nach Anspruch 1, dadurch gekennzeichnet, daß man das Kupfer(I)-chlorid in Mengen von 2 bis 5 Mol-%, bezogen auf Retinol einsetzt.

4. Verfahren gemäß Anspruch 1, dadurch gekennzeichnet, daß man als Amidgruppen enthaltendes Lösungsmittel N,N-Dimethylformamid, N,N-Dimethyl-acetamid, 1-Methyl-2-pyrrolidon, N,N-Dimethyl-propylenharnstoff oder N,N-Dimethyl-ethylenharnstoff verwendet.

5. Verfahren gemäß Anspruch 1, dadurch gekennzeichnet, daß man als Lösungsmittel Dimethylformamid verwendet.

## Claims

1. A process for preparing retinal, which comprises oxidizing retinol with oxygen or oxygen-containing gases in homogeneous phase in a solvent containing amide groups with catalytic amounts of 4-hydroxy-2,2,6,6-tetramethyl-1-piperidinyl oxide and catalytic amounts of copper(I) chloride.

2. A process as claimed in claim 1, wherein the 4-hydroxy-2,2,6,6-tetramethyl-1-piperidinyl oxide is employed in amounts of from 2 to 5 mol%, based on retinol.

3. A process as claimed in claim 1, wherein the copper(I) chloride is employed in amounts of from 2 to 5 mol%, based on retinol.

4. A process as claimed in claim 1, wherein N,N-dimethylformamide, N,N-dimethylacetamide, 1-methyl-2-pyrrolidone, N,N-dimethylpropyleneurea or N,N-dimethylethyleneurea is used as solvent containing amide groups.

5. A process as claimed in claim 1, wherein dimethylformamide is used as solvent.

## Revendications

1. Procédé pour la préparation du rétinal, caractérisé par le fait que l'on oxyde le rétinol en phase homogène dans un solvant contenant des groupes amide, en présence de quantités catalytiques de 4-hydroxy-2,2,6,6-tétraméthyl-pipéridine-1-oxyle et de quantités catalytiques de chlorure cuivreux, par l'oxygène ou des gaz contenant de l'oxygène.

2. Procédé selon la revendication 1, caractérisé par le fait que l'on utilise le 4-hydroxy-2,2,6,6-tétraméthyl-pipéridine-1-oxyle en quantité de 2 à 5 mol % par rapport au rétinol.

3. Procédé selon la revendication 1, caractérisé par le fait que l'on utilise le chlorure cuivreux en quantité de 2 à 5 mol pour 100 mol de rétinol.

4. Procédé selon la revendication 1, caractérisé par le fait que l'on utilise, en tant que solvant contenant des groupes amide, le N,N-diméthylformamide, le N,N-diméthylacétamide, la 1-méthyl-2-pyrrolidone, la N,N-diméthylpropylèneurée ou la N,N-diméthyléthylèneurée.

5. Procédé selon la revendication 1, caractérisé par le fait que le solvant utilisé est le diméthylformamide.
